# EUROPÄISCHE PATENTSCHRIFT

(11) **EP 4 373 418 B1**
(45) Veröffentlichungstag und Bekanntmachung des Hinweises auf die Patenterteilung: **08.04.2026**
(21) Anmeldenummer: 22797765.9
(22) Anmeldetag: 05.10.2022
(51) Int. Cl.: A61B 17/80

(54) **PLATTENIMPLANTAT**
PLATE IMPLANT
IMPLANT DE TYPE PLAQUE

(30) Priorität: 05.10.2021 DE 202021003115 U
(43) Veröffentlichungstag der Anmeldung: 29.05.2024
(73) Patentinhaber: Karl Leibinger Asset Management GmbH & Co. KG, 78570 Mühlheim an der Donau (DE)
(72) Erfinder: MOSLER, Lüder, 37115 Duderstadt (DE)
(74) Vertreter: Isarpatent
(86) Internationale Anmeldenummer: PCT/EP2022/077628
(87) Internationale Veröffentlichungsnummer: WO 2023/057477

(56) Entgegenhaltungen:
- DE-A1- 19 855 254
- DE-A1- 2 438 669
- DE-A1- 3 912 703
- US-A1- 2012 277 748

## Beschreibung

Die vorliegende Erfindung betrifft ein Plattenimplantat zur Fixierung von Frakturen.

Die DE 198 55 254 A1 offenbart eine Vorrichtung zur Retention und Protektion von beschädigten Knochen, welche zwei beiderseits der Knochenbeschädigung an einem Knochen fixierbare Abschnitte aufweist, welche zur Überbrückung der Knochenbeschädigung über mindestens eine Federeinrichtung axial federnd gegeneinander verschiebbar miteinander gekoppelt sind. Des Weiteren besitzt die Vorrichtung Mittel zum Variieren der Federkonstante.

Die DE 39 12 703 A1 offenbart eine Fixierungsvorrichtung für Röhrenknochenfrakturen, bestehend aus einem langgestreckten, abgeflachten Fixierungselement mit im Wesentlichen rechteckigem Querschnitt aus gewebekompatiblem hochfesten Material, insbesondere Implantatstahl, in dessen Endabschnitten jeweils mindestens zwei sich durch seine gegenüberliegenden breiten Seiten erstreckende Bohrungen ausgebildet sind, und aus durch diese Bohrungen geführten Knochenschrauben. Das Fixierungselement weist zwischen den die Bohrungen für die Knochenschrauben aufweisenden steifen Endabschnitten einen elastischen Verbindungsabschnitt mit einem Querschnitt auf, der kleiner ist als der der Endabschnitte.

Die US 2012/277748 A1 offenbart eine Platte zum Verbinden eines ersten Knochenabschnitts und eines zweiten Knochenabschnitts. Die Platte umfasst eine zentrale Längsachse, einen ersten Endabschnitt, der sich entlang der zentralen Längsachse erstreckt und zur Befestigung am ersten Knochenabschnitt konfiguriert ist, einen zweiten Endabschnitt, der sich entlang der zentralen Längsachse erstreckt und zur Befestigung am zweiten Knochenabschnitt konfiguriert ist, und einen Zwischenabschnitt, der sich entlang der zentralen Längsachse und zwischen dem ersten Endabschnitt und dem zweiten Endabschnitt erstreckt. Der Zwischenabschnitt umfasst drei durchgehende Materialsegmente, von denen sich zwei Segmente entlang einer Richtung erstrecken, die im Wesentlichen parallel zur zentralen Längsachse ist, und von denen sich ein Segment entlang einer Richtung erstreckt, die im Wesentlichen senkrecht zur zentralen Längsachse ist.

Die DE 24 38 669 A1 offenbart eine Osteosyntheseplatte mit einem länglichen Körper, der mehrere, in Längsrichtung des Körpers beanstandete und zum Einsetzen von Knochenschrauben dienende Löcher aufweist, von denen mindestens eines durch ein mit dem Rest des Körpers über ein stabartiges Teil verbundenes Auge gebildet ist, wobei das stabartige Teil federelastisch ist, quer zur Längsrichtung des Körpers verläuft und eine Verlagerung des Auges in Längsrichtung des Körpers gestattet.

Zur Fixierung von Frakturen während der Knochenheilung werden häufig Implantate in Form von Platten verwendet (sogenannte Plattenimplantate bzw. Osteosyntheseplatten). Diese flachen, in der Regel langestreckten Plattenimplantate sind beispielsweise mit einer Vielzahl von Bohrungen für Knochenschrauben versehen, mittels derer sie mit dem Knochen verschraubt werden. Sie sorgen für eine Beruhigung des Frakturspaltes während der Knochenheilung und werden nach gutem Heilungsverlauf in der Regel operativ entfernt.

In der Praxis wird beobachtet, dass die Knochenheilung bei Versorgung mit Plattenimplantaten verzögert oder unvollständig erfolgt. Dieses wird darauf zurückgeführt, dass ein gewisses Maß an Bewegung im Knochenspalt erforderlich ist, um die Knochenheilung zu stimulieren. Die medizinische Literatur nennt hierzu axiale Bewegungen im Bereich weniger Zehntel-Millimeter bei gleichzeitiger Unterdrückung von Scher- und Torsionsbewegungen.

Die WO 2021/102591 A1 beschreibt ein Plattenimplantat mit Einschnitten im Bereich des Frakturspalts, die eine begrenzte Beweglichkeit zulassen. Die Einschnitte reduzieren den aststragenden Querschnitt des Plattenimplantats im Bereich des Frakturspalts auf eine dünne Blattfeder, deren Biegung und Längsdehnung durch Anschläge begrenzt wird. Konstruktionsbedingt lässt das Implantat ein begrenztes Verschwenken der fixierten Knochen, aber keine längsaxiale Bewegung derselben zu.

Die EP 1 221 308 A1 offenbart ein Wirbelsäulen-Implantat mit Blattfedern, die der Reduktion der Biegesteifigkeit des Implantats dienen. Eine verstärkte Längsdehnung des Implantats wird durch die Blattfedern nicht unterstützt.

Der vorliegenden Erfindung liegt folglich die Aufgabe zugrunde, ein Plattenimplantat zu konzipieren, das Frakturspalte überbrückt und stabilisiert, aber dennoch eine begrenzte Bewegung in Richtung der (Knochen)-Längsachse zulässt.

Die Erfindung schafft ein Plattenimplantat nach Anspruch 1.

Dem erfindungsgemäßen Plattenimplantat liegt die Idee zugrunde, eine elastische Längsdehnungseinrichtung vorzusehen, welche zumindest zwischen zwei Bohrungen vorgesehen ist. Die Längsdehnungseinrichtung ist derart gestaltet, dass das Plattenimplantat eine erhöhte lokale Dehnbarkeit in Richtung der Knochen-Längsachse bei gleichzeitig hoher Biege- und Torsionssteifigkeit aufweist.

Dies ermöglicht, ein gewisses Maß an Längsbewegung im Frakturspalt, um die Knochenheilung zu stimulieren.

Erfindungsgemäß weist die Längsdehnungseinrichtung mehrere Blattfedern auf, welche durch einen oder mehrere Einschnitte im Plattenimplantat gebildet ist.

Erfindungsgemäß sind die Blattfedern durch u-förmige Einschnitte im Plattenimplantat gebildet, welche eine jeweilige Bohrung einschließen.

Gemäß einer weiteren bevorzugten Weiterbildung ist eine erste Mehrzahl von Einschnitten derart gestaltet, dass die u-Form zum linken Rand des Plattenimplantats hin geöffnet ist, und eine zweite Mehrzahl von Einschnitten derart gestaltet, die u-Form zum rechten Rand des Plattenimplantats hin geöffnet ist.

Gemäß einer weiteren bevorzugten Weiterbildung überlappen sich die Einschnitte lateral.

Gemäß einer weiteren bevorzugten Weiterbildung sind die Blattfedern derart gestaltet, dass sie durch jeweilige erste, zweite und dritte Einschnitte gebildet sind, wobei die ersten und zweiten Einschnitte eine jeweilige Bohrung zumindest teilweise u-förmig umgeben, wobei die erste u-Form zum linken Rand des Plattenimplantats hin geöffnet ist und die zweite u-Form zum rechten Rand des Plattenimplantats hin geöffnet ist und wobei die dritten Einschnitte jeweils senkrecht zur Längsachse angeordnet sind und zwischen und beabstandet von jeweiligen Paaren der ersten und zweiten Einschnitte angeordnet sind.

Gemäß einer weiteren bevorzugten Weiterbildung sind die zweiten Einschnitte von den ersten Einschnitten gegenüberliegend beabstandet sind, wobei die dritten Einschnitte jeweils senkrecht zur Längsachse angeordnet sind zwischen und beabstandet von jeweiligen Paaren ersten und zweiten Einschnitte derart angeordnet sind, dass die Blattfedern ausgebildet sind.

Gemäß einer weiteren bevorzugten Weiterbildung weist das Plattenimplantat einen Rumpfbereich mit einer im Wesentlichen konstanten ersten Breite sowie einen sich von der ersten Breite kontinuierlich verbreiternden Kopfbereich auf.

Die Erfindung wird nachfolgend anhand bevorzugter Ausführungsformen mit Bezug auf die Figuren näher erläutert.

Es zeigen:
- Fig. 1: ein beispielhaftes Plattenimplantat, welches nicht Gegenstand der vorliegenden Erfindung ist;
- Fig. 2: ein Plattenimplantat gemäß einer ersten Ausführungsform der vorliegenden Erfindung;
- Fig. 3: ein Plattenimplantat gemäß einer zweiten Ausführungsform der vorliegenden Erfindung;
- Fig. 4: ein Plattenimplantat gemäß einer dritten Ausführungsform der vorliegenden Erfindung;
- Fig. 5: ein Plattenimplantat gemäß einer vierten Ausführungsform der vorliegenden Erfindung;
- Fig. 6: ein Plattenimplantat gemäß einer fünften Ausführungsform der vorliegenden Erfindung; und
- Fig. 7: ein Plattenimplantat gemäß einer sechsten Ausführungsform der vorliegenden Erfindung.

Im Folgenden sind gleiche oder funktionsgleiche Elemente mit gleichen Bezugszeichen versehen.

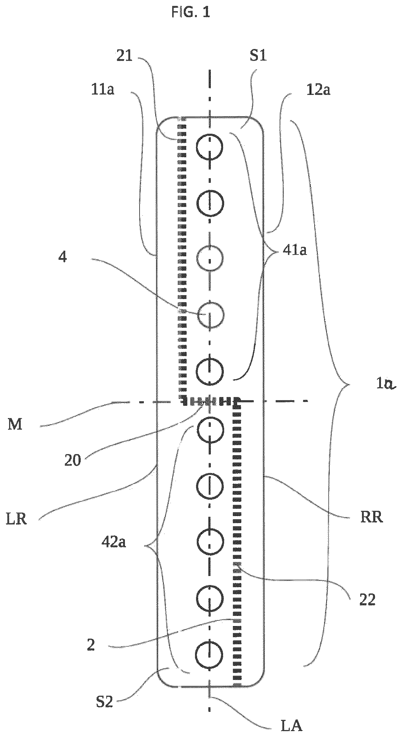

Fig. 1 zeigt ein beispielhaftes Plattenimplantat, welches nicht Gegenstand der vorliegenden Erfindung ist.

Das längliche Plattenimplantat 1a weist eine erste und eine zweite Teilplatte 11a, 12a auf, welche über eine Längsdehnungseinrichtung in Form eines elastischen Elements 2 miteinander verbunden sind. Eine Mehrzahl von zehn Bohrungen 4 verläuft entlang einer zentralen Längsachse LA des Plattenimplantats 1a, wobei bezüglich einer Mittelquerachse M des Plattenimplantats fünf erste Bohrungen 41a auf einer ersten Seite S1 der Mittelquerachse M und fünf zweite Bohrungen 42a auf der anderen zweiten Seite S2 der Mittelquerachse M angeordnet sind. Das elastische Element 2 verläuft in einem ersten geradlinigen Abschnitt 21 auf der ersten Seite S1 versetzt gegenüber den ersten Bohrungen 41a zum linken Rand LR des Plattenimplantats 1a hin und parallel zu den ersten Bohrungen 41a, und in einem zweiten geradinigen Abschnitt 22 auf der zweiten Seite S2 versetzt gegenüber den zweiten Bohrungen 42a zum rechten Rand RR des Plattenimplantats 1a hin und parallel zu den zweiten Bohrungen 42a. Entlang der Mittelquerachse M sind der erste Abschnitt 21 und der zweite Abschnitt 22 durch einen dritten geradlinigen Abschnitt 20 des elastischen Elements 2 miteinander verbunden.

Operativ wird der Frakturspalt entlang der Mittelquerachse M angeordnet und werden die beiden Teilplatten 11a, 12a mittels Fixierelementen (nicht dargestellt), z.B. Schrauben, an den beiden durch den Frakturspalt getrennten Knochenfragmenten angebracht. Welche Bohrungen 4 für die Fixierelemente verwendet werden, richtet sich nach Art und Verlauf des Frakturspalts.

Bei Druck oder Zug auf den Frakturspalt kann sich das Plattenimplantat 1a in Richtung der zentralen Längsachse LA elastisch verformen, ohne wesentlich an Steifigkeit in Biege-und Torsionsrichtung einzubüßen.

Das elastische Element 2 kann beispielsweise als Elastomereinlage gestaltet sein, welche keine Biege-und Torsionsbewegungen zulässt, wohl aber eine Längsverformung.

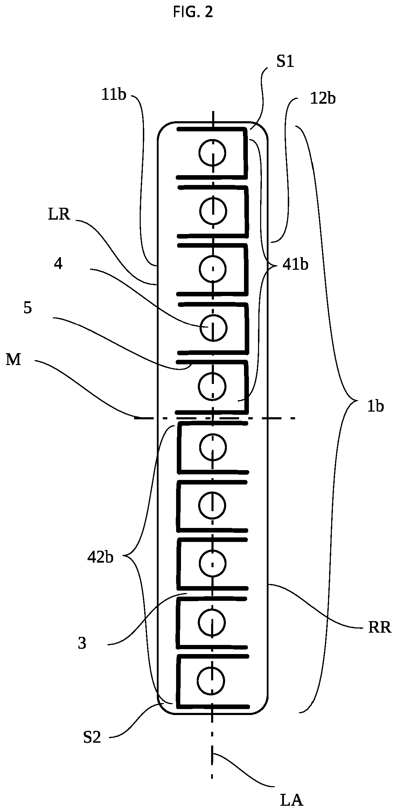

Fig. 2 zeigt ein Plattenimplantat gemäß einer ersten Ausführungsform der vorliegenden Erfindung.

Das längliche Plattenimplantat 1b gemäß Fig. 2 weist eine erste und eine zweite Teilplatte 11b, 12b auf, welche links und rechts der zentralen Längsachse LA des Plattenimplantats 1b angeordnet sind. Die erste und zweite Teilplatte 11b, 12b weisen eine Längsdehnungseinrichtung in Form einer Mehrzahl von Blattfedern 3 auf.

Eine Mehrzahl von zehn Bohrungen 4 verläuft entlang der zentralen Längsachse LA des Plattenimplantats 1b, wobei bezüglich einer Mittelquerachse M des Plattenimplantats fünf erste Bohrungen 41b auf einer ersten Seite S1 der Mittelquerachse M und fünf zweite Bohrungen 42b auf der anderen zweiten Seite S2 der Mittelquerachse M angeordnet sind.

Die Blattfedern 3 verlaufen senkrecht zur zentralen Längsachse LA des Plattenimplantats 1b. Sie ermöglichen ein elastisches Einfedern in Richtung der zentralen Längsachse LA.

Die Blattfedern 3 sind durch u-förmige Einschnitte 5 im Plattenimplantat 1b verwirklicht, welche eine jeweilige Bohrung 4 einschließen. Insbesondere sind die Einschnitte 5 auf der ersten Seite S1 der Mittelquerachse M derart vorgesehen, dass die u-Form zum linken Rand LR hin geöffnet ist, und die Einschnitte 5 auf der zweiten Seite S2 der Mittelquerachse M sind derart vorgesehen, dass die u-Form zum rechten Rand RR hin geöffnet ist.

Die Teilplatten 11b, 12b und die Blattfedern 3 bilden somit eine Leiterstruktur, die sich aufgrund der Elastizität der Blattfedern parallelogramm-artig scheren und somit in Längsrichtung dehnen lässt.

Operativ kann der Frakturspalt entlang der Mittelquerachse M angeordnet werden, und die beiden Teilplatten 11b, 12b werden mittels Fixierelementen (nicht dargestellt), z.B. Schrauben, an den beiden durch den Frakturspalt getrennten Knochenfragmenten angebracht. Allerdings ist es bei dieser zweiten Ausführungsform aufgrund der Symmetrie möglich, dass der Frakturspalt versetzt zur Mittelquerachse M im Bereich einer Blattfeder angeordnet wird. Die Bohrungen 4 der Teilplatten 11b, 12b sind dann je nach Ort des Frakturspaltes jeweils der Teilplatte 11b oder der Teilplatte 12b zugeordnet.

Bei Druck oder Zug auf den Frakturspalt kann sich das Plattenimplantat 1b folglich in Richtung der zentralen Längsachse LA elastisch verformen, ohne wesentlich an Steifigkeit in Biege-und Torsionsrichtung einzubüßen. Die Summe der Biegesteifigkeiten der Blattfedern 3 ergibt die Gesamtsteifigkeit des Plattenimplantats 1b für die gewünschte Bewegung. Die höchsten Kräfte quer zur Blattfederfläche sind in der Nähe des Ortes des Frakturspaltes zu erwarten. Es ist somit sinnvoll die Dicke der Blattfedern 3 in der Nähe des Ortes des Frakturspaltes stärker bzw. breiter auszuführen.

Die Einschnitte 5 können durch Verfahren wie Laserstrahlschneiden, Wasserstrahlschneiden oder Erodieren erzeugt werden. Die Schnittbreite kann geeignet gewählt werden, um die Bewegung in Richtung der zentralen Längsachse LA zu begrenzen. Alternativ kann das Plattenimplantat 1b auch additiv, z.B durch Lasersintern, gefertigt werden. Hierbei kann nicht nur die Topolgie des Knochens, sondern auch die Lage des Frakturspaltes besser berücksichtigt werden. Als Material für das Plattenimplantat kommen die üblichen korrosionsfesten Stähle oder Titan oder Titanlegierungen in Frage.

Speziell bei einer individuellen Anfertigung eines solchen Plattenimplantates 1b wäre es alternativ möglich die Orientierung der Blattfedern so abzuändern, dass die bei Belastung entstehende Bewegung in etwa senkrecht zur Fläche des Frakturspaltes erfolgt. Hierzu verliefe die Fläche der Blattfedern vorzugsweise in etwa parallel zum Frakturspalt.

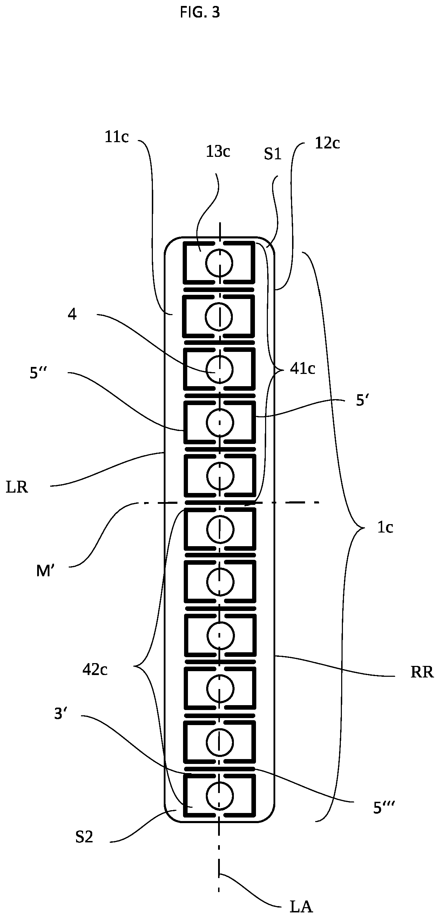

Fig. 3 zeigt ein Plattenimplantat gemäß einer zweiten Ausführungsform der vorliegenden Erfindung.

Fig. 3 zeigt ein alternatives Plattenimplantat 1c mit einer ersten und zweiten Teilplatte 11c, 12c sowie einer Mehrzahl dritter Teilplatten 13c. Wie bei den vorhergehenden Ausführungsformen wird der Ort des Frakturspalt von den Teilplatten 11,c, 12c, 13c überbrückt, beispielsweise im Bereich der Mittelquerachse oder, wie dargestellt entlang einer bezüglich der Mittelquerachse verschobenen Achse M'. Vorteilhaft bei dieser Ausführungsform ist somit ebenfalls, dass die Lage des Frakturspaltes relativ zum Plattenimplantat 1c nicht eng festgelegt ist.

Bezüglich der verschobenen Achse M' des Plattenimplantats 1c sind fünf erste Bohrungen 41c auf einer ersten Seite S1 der Achse M' und sechs zweite Bohrungen 42c auf der anderen zweiten Seite S2 der Achse M' angeordnet
Die Teilplatten 13c sind entlang der zentralen Längsachse LA mit Blattfedern 3' als Längsdehnungseinrichtung verbunden, die jeweils paarweise ausgebildet sind und die weiteren Teilplatten 11c, 12c haltern. Die dritten Teilplatten 13c sind jeweils mit einer Bohrung 4 entlang der zentralen Längsachse LA versehen.

Die Blattfedern 3' sind derart gestaltet, dass sie durch jeweilige erste, zweite und dritte Einschnitte 5', 5", 5‴ gebildet werden, wobei die ersten Einschnitte 5' die dritten Teilplatten 13c auf der ersten Teilplatte 11c u-förmig abgrenzen und die jeweilige Bohrung 4 teilweise umgeben. Die zweiten Einschnitte 5" sind derart gebildet, dass sie die dritten Teilplatten 13c auf der zweiten Teilplatte 12c u-förmig abgrenzen und die jeweilige Bohrung 4 teilweise umgeben, wobei sie von den ersten Einschnitten 5' beabstandet sind.

Die dritten Einschnitte 5‴ sind jeweils senkrecht zur zentralen Längsachse LA angeordnet und liegen zwischen und beabstandet von jeweiligen Paaren ersten und zweiten Einschnitte 5', 5".

Die Teilplatten 13c weisen hierdurch eine Nachgiebigkeit in Richtung der Längsachse auf, sind aber in Richtung der Bohrungsachse und quer zur Längsachse LA steif gelagert.

Es müssen nicht alle Bohrungen 4 elastisch gelagert werden. Ein solches Implantat ist z.B. geeignet um Frakturen in der Nähe von Gelenken oder komplexere Frakturen zu versorgen.

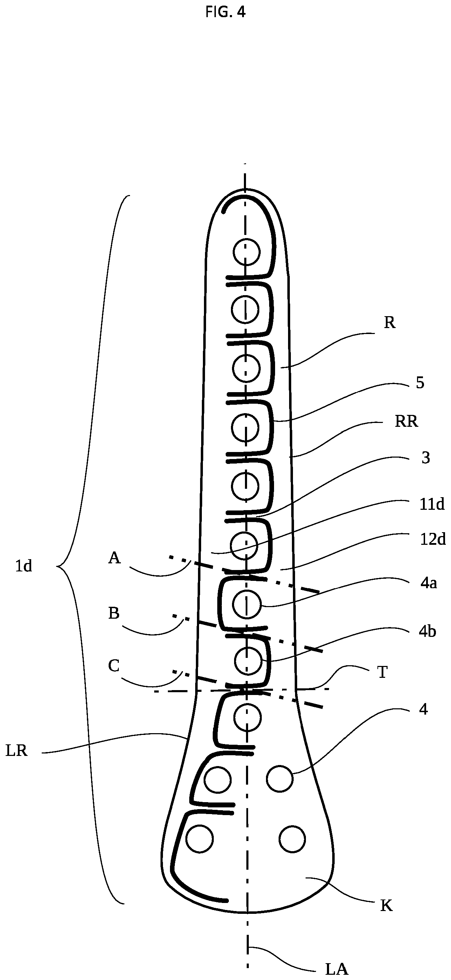

Fig. 4 zeigt ein Plattenimplantat gemäß einer dritten Ausführungsform der vorliegenden Erfindung.

Das längliche Plattenimplantat 1d gemäß Fig. 4 kann beispielsweise für die Versorgung von distalen Tibia-Frakturen Verwendung finden. Es weist einen Rumpfbereich R mit einer im Wesentlichen konstanten ersten Breite sowie einen sich von der ersten Breite kontinuierlich verbreiternden Kopfbereich K auf (virtuelle Trennlinie T).

Die Teilplatten 11d, 12d erstrecken sich jeweils über die gesamte Länge des Plattenimplantats 1d. Wie bei der zweiten Ausführungsform weisen die erste und eine zweite Teilplatte 11d, 12d eine Längsdehnungseinrichtung in Form einer Mehrzahl von Blattfedern 3 auf. Eine Mehrzahl von 13 Bohrungen ist in dem Plattenimplantat 1d vorgesehen.

Im oberen proximalen Rumpfbereich R und am Beginn des unteren distalen Kopfbereichs K sind neun Bohrungen 4 entlang der zentralen Längsachse LA angeordnet. Im weiteren Verlauf des unteren distalen Kopfbereichs K sind jeweils zwei Bohrungen 4 der linken bzw. rechten Teilplatte 11d, 12d zugeordnet bzw. und dort zum rechten Rand RR bzw. linken Rand LR hin versetzt angeordnet.

Die Blattfedern 3 ermöglichen ein elastisches Einfedern in Richtung der zentralen Längsachse LA und sind durch u-förmige Einschnitte 5 im Plattenimplantat 1d verwirklicht, welche eine jeweilige Bohrung 4 im Rumpfbereich R entlang der zentralen Längsachse LA einschließen. Im weiteren Verlauf des oberen, proximalen Kopfbereichs K sind jeweils zwei Bohrungen 4 der linken bzw. rechten Teilplatte 11d, 12d zugeordnet, und die Blattfedern 3 sind zum linken Rand LR hin versetzt und weisen eine asymmetrische u-Form auf.

Insbesondere sind die ersten sechs Einschnitte 5 und der achte Einschnitt 5 im Rumpfbereich R derart vorgesehen, dass die u-Form zum linken Rand LR hin geöffnet ist, wohingegen der siebte Einschnitt 5 im Rumpfbereich R derart vorgesehen ist, dass die u-Form zum rechten Rand RR hin geöffnet ist. Auch die Einschnitte 5 im Kopfbereich K sind derart vorgesehen, dass die u-Form zum rechten Rand RR hin geöffnet ist.

Die Teilplatten 11b, 12b und die Blattfedern 3 bilden eine somit Leiterstruktur, die sich aufgrund der Elastizität der Blattfedern 3 eine Längsdehnungseinrichtung bilden, die sich parallelogramm-artig scheren lässt.

Die wechselseitige Anordnung der Blattfedern 3 ermöglicht es, durch Wahl der Verschraubungen in den Bohrungen 4 dem Ort des Frakturspaltes oder der Frakturspalte gerecht zu werden. Gerade den mittleren Verschraubungen kommt eine große Bedeutung bei der Stabilisierung der Fraktur gegen Biegebelastungen zu, so dass es wichtig ist gerade in diesem Bereich Schrauben einsetzen zu können.

Der Ort des Frakturspaltes ist in den alternativen Lagen A, B oder C angedeutet. Läge der Ort des Frakturspaltes beispielsweise bei A, würde in Bohrung 4a eine Schraube gesetzt werden, Bohrung 4b hingegen bliebe frei. Läge der Ort des Frakturspaltes beispielsweise bei B, würden weder in Bohrung 4a noch in Bohrung 4b eine Schraube gesetzt werden. Läge der Ort des Frakturspaltes beispielsweise bei C, würde in Bohrung 4b eine Schraube gesetzt werden, Bohrung 4a hingegen bliebe frei.

Ebenso ist in Fig. 4 erkennbar, dass die Blattfedern 3 an ihren Enden weiche Übergänge zu den Teilplatten 11d, 12d aufweisen. Dieses verbessert aufgrund der verringerten Kerbwirkung die Schwingfestigkeit des Plattenimplantats 1d was insofern von Bedeutung ist, weil die für Implantate üblichen Werkstoffe eine gewisse Kerbempfindlichkeit aufweisen. Ebenfalls erkennbar ist, dass die resultierende Breite der Teilplatten im Umfeld des Ortes des Frakturspaltes zunimmt, um den voraussichtlich auftretenden größeren Biegemomenten in diesem Bereich Rechnung zu tragen.

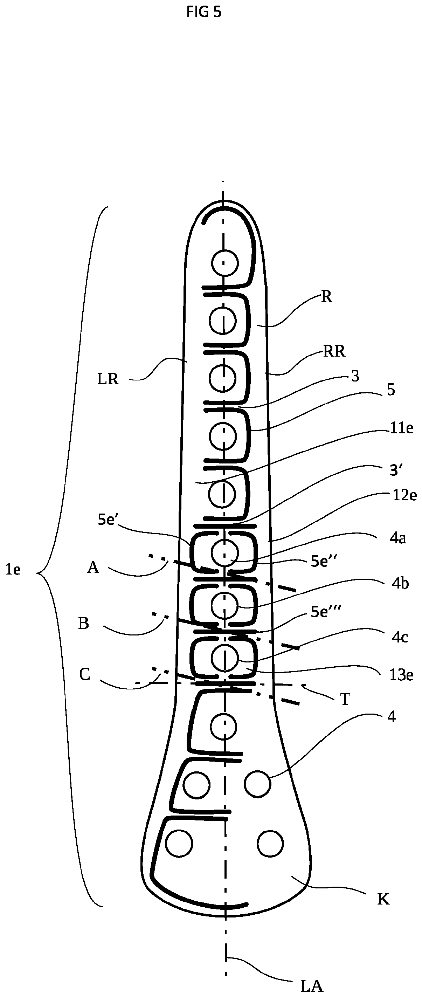

Fig. 5 zeigt ein Plattenimplantat gemäß einer vierten Ausführungsform der vorliegenden Erfindung, wie es beispielsweise für die Versorgung von distalen Tibia-Frakturen Verwendung finden könnte.

Das Plattenimplantat 1e mit einer ersten und zweiten Teilplatte 11e, 12e sowie einer Mehrzahl dritter Teilplatten 13e unterscheidet sich darin von derjenigen des Plattenimplantats 1d der vierten Ausführungsform, dass die Blattfedern 3' um die sechste bis achte Bohrung 4 analog ausgebildet sind zu den Blattfedern 3' der dritten Ausführungsform.

Insbesondere sind diese Blattfedern 3' derart gestaltet, dass sie durch jeweilige erste, zweite und dritte Einschnitte 5e', 5e", 5e‴ gebildet werden, wobei die ersten Einschnitte 5e' die dritten Teilplatten 13e auf der ersten Seite S1 u-förmig abgrenzen und die jeweilige Bohrung 4 teilweise umgeben. Die zweiten Einschnitte 5e" sind derart gebildet, dass sie die dritten Teilplatten 13c auf der zweiten Seite S2 u-förmig abgrenzen und die jeweilige Bohrung 4 teilweise umgeben, wobei die von den ersten Einschnitten 5e' beabstandet sind. Die dritten Einschnitte 5e‴ sind jeweils senkrecht zur zentralen Längsachse LA angeordnet und liegen zwischen und beabstandet von jeweiligen Paaren ersten und zweiten Einschnitte 5e', 5e".

Die Teilplatten 13e weisen hierdurch eine Nachgiebigkeit in Richtung der Längsachse auf, sind aber in Richtung der Bohrungsachse und quer zur Längsachse LA steif gelagert.

Diese Anordnung ermöglicht es ebenfalls, unabhängig von der Lage des Ortes des Frakturspaltes Schrauben in dessen Nähe entlang der Lagen A, B, C zu setzten, ohne die Bewegung im Frakturspalt dadurch einzuschränken. Somit können die Bohrungen 4a, 4b, 4c abhängig vom Frakturspalt mit Fixierelementen belegt werden. Somit ist diese Variante ebenfalls vorteilhaft bei der Fixierung von Fragmenten.

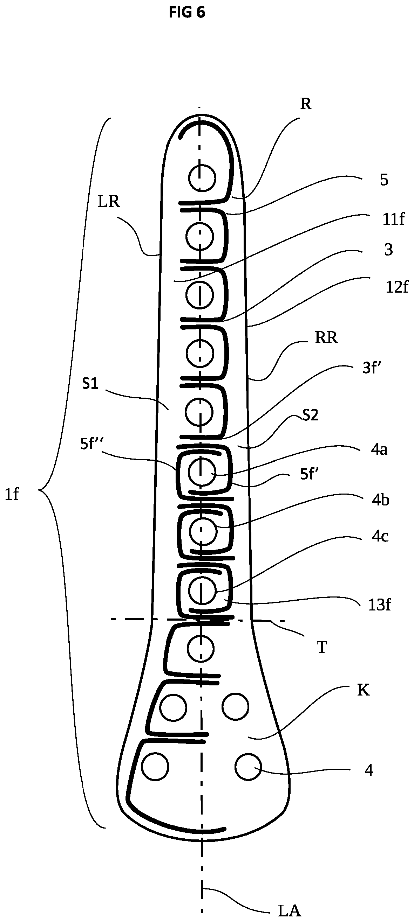

Fig. 6 zeigt ein Plattenimplantat gemäß einer fünften Ausführungsform der vorliegenden Erfindung.

Das Plattenimplantat 1f mit einer ersten und zweiten Teilplatte 11f, 12f sowie einer Mehrzahl dritter Teilplatten 13f unterscheidet sich darin von derjenigen des Plattenimplantats 1d der vierten Ausführungsform, dass die Blattfedern 3f um die sechste bis achte Bohrung 4 derart gestaltet sind, dass die Aufhängung der Teilplatten 13f mit nur zwei Einschnitten 5f', 5f" vergrößerter Länge beabstandet überlappend gebildet sind. Daraus folgt eine vergrößerte Nachgiebigkeit.

Insbesondere sind diese Blattfedern 3f derart gestaltet, dass sie durch jeweilige erste und zweite Einschnitte 5f', 5ef" gebildet werden, wobei die ersten Einschnitte 5f' die dritten Teilplatten 13f auf der ersten Seite S1 u-förmig abgrenzen und die jeweilige Bohrung 4 teilweise überlappend umgeben. Die zweiten Einschnitte 5f" sind derart gebildet, dass sie die dritten Teilplatten 13f auf der zweiten Seite S2 u-förmig abgrenzen und die jeweilige Bohrung 4 teilweise umgeben, wobei die von den ersten Einschnitten 5f' beabstandet sind.

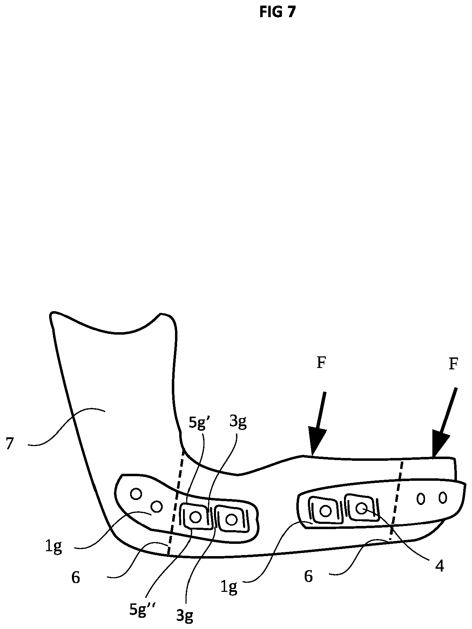

Fig. 7 zeigt ein Plattenimplantat gemäß einer sechsten Ausführungsform der vorliegenden Erfindung bei Anwendung bei einer Rekonstruktion eines Unterkiefers 7 aus Fragmenten der Fibula.

Fig. 7 zeigt die Anwendung zweier Plattenimplantate 1g, 1g' mit Blattfedern 3g und Einschnitten 5g', 5g" analog zu den Blattfedern 3f und Einschnitten 5f', 5f" gemäß Fig. 6 bei einer Rekonstruktion eines Unterkiefers aus Fragmenten der Fibula. Eingesetzt werden individuell gestaltete Plattenimplantate 1g, 1g', die jeweils Orte des jeweiligen Frakturspaltes 6 überbrücken.

Die Funktion besteht darin, statt einer starren Fixierung der Fragmente durch Dynamisierung aufgrund der wirkenden Kräfte F auf Abschnitte des rekonstruierten Unterkiefers 7 Bewegungen und Kräfte zu erzeugen, die zu einer Kompression der Frakturspalte beitragen.

Dieses wird ermöglicht, indem die Blattfedern 3g leicht gewinkelt zum Ort des Frakturspaltes orientiert sind. Es entsteht eine Komponentkraft die den Frakturspalt schließt.

## Patentansprüche

1. Plattenimplantat (1b-1g) zur Überbrückung eines Frakturspalts (6) mit:
einer Längsachse (LA) in Richtung der größten Ausdehnung des Plattenimplantats (1b-1g);
einer Mehrzahl von Bohrungen (4), welche voneinander beabstandet in Richtung der Längsachse (LA) angeordnet sind; und
einer elastischen Längsdehnungseinrichtung (3; 3'; 3, 3'; 3, 3f; 3g), welche zumindest zwischen zwei Bohrungen (4) vorgesehen ist;
wobei die Längsdehnungseinrichtung (3; 3'; 3, 3'; 3, 3f; 3g) derart gestaltet ist, dass das Plattenimplantat (1b-1g) eine erhöhte lokale Dehnbarkeit in Richtung der Knochen-Längsachse (LA) bei gleichzeitig hoher Biege- und Torsionssteifigkeit aufweist;
wobei die Längsdehnungseinrichtung (3; 3'; 3, 3'; 3, 3f; 3g) mehrere Blattfedern (3; 3'; 3, 3'; 3, 3f; 3g) aufweist, welche durch einen oder mehrere Einschnitte (5; 5', 5", 5"; 5; 5, 5e', 5e", 5e‴; 5, 5f', 5f"; 5g', 5g") im Plattenimplantat (1b-g) gebildet sind; und
wobei die Blattfedern (3; 3'; 3, 3'; 3, 3f; 3g) durch u-förmige Einschnitte (5; 5', 5", 5"; 5; 5, 5e', 5e", 5e‴; 5, 5f', 5f"; 5g', 5g") im Plattenimplantat (1b-1g) gebildet sind, welche eine jeweilige Bohrung (4) einschließen.

2. Plattenimplantat (1b-g) zur Überbrückung eines Frakturspalts (6) nach Anspruch 1, wobei eine erste Mehrzahl von Einschnitten (5f', 5f"; 5g', 5g") derart gestaltet ist, dass die u-Form zum linken Rand (LR) des Plattenimplantats hin geöffnet ist, und eine zweite Mehrzahl von Einschnitten (5, 5f', 5f"; 5g', 5g") derart gestaltet ist, die u-Form zum rechten Rand (RR) des Plattenimplantats hin geöffnet ist.

3. Plattenimplantat (1b-g) zur Überbrückung eines Frakturspalts (6) nach Anspruch 1, wobei sich die Einschnitte (5f', 5f"; 5g', 5g") lateral überlappen.

4. Plattenimplantat (1b-g) zur Überbrückung eines Frakturspalts (6) nach Anspruch 1, wobei die Blattfedern (3') derart gestaltet sind, dass sie durch jeweilige ersteund zweite Einschnitte (5', 5") gebildet sind, wobei die ersten und zweiten Einschnitte (5") eine jeweilige Bohrung (4) zumindest teilweise u-förmig umgeben, wobei die u-Form der ersten Einschnitte (5') zum linken Rand (LR) des Plattenimplantats hin geöffnet ist und die u-Form der zweiten Einschnitte (5") zum rechten Rand (RR) des Plattenimplantats hin geöffnet ist, und wobei weitere Blattfedern (3') durch dritte Einschnitte (5‴) gebildet sind, welche jeweils senkrecht zur Längsachse (LA) angeordnet sind und zwischen und beabstandet von jeweiligen Paaren der ersten und zweiten Einschnitte (5', 5") angeordnet sind.

5. Plattenimplantat (1ba-g) zur Überbrückung eines Frakturspalts (6) nach Anspruch 4, wobei die zweiten Einschnitte (5") von den ersten Einschnitten (5') gegenüberliegend beabstandet sind, und wobei die dritten Einschnitte (5‴) jeweils senkrecht zur Längsachse (LA) zwischen und beabstandet von jeweiligen Paaren der ersten und zweiten Einschnitte (5', 5")angeordnet sind.

6. . Plattenimplantat (1b-g) zur Überbrückung eines Frakturspalts (6) nach einem der vorhergehenden Ansprüche, **gekennzeichnet durch** einen Rumpfbereich (R) mit einer im Wesentlichen konstanten ersten Breite sowie einen sich von der ersten Breite kontinuierlich verbreiternden Kopfbereich (K).

7. Plattenimplantat (1g, 1g') nach Anspruch 1, **dadurch gekennzeichnet, dass** das Plattenimplantat (1g, 1g') individuell gestaltet und zur Rekonstruktion eines Unterkiefers (7) aus Fragmenten einer Fibula vorgesehen ist.

8. Set aus zwei Plattenimplantaten (1g, 1g') gemäß Anspruch 7 zur Rekonstruktion eines Unterkiefers (7) aus Fragmenten der Fibula.

## Claims

1. Plate implant (1b-1g) for bridging a fracture gap (6) having:
a longitudinal axis (LA) in the direction of the greatest extension of the plate implant (1b-1g);
a plurality of holes (4), which are spaced apart from one another in the direction of the longitudinal axis (LA); and
an elastic longitudinal expansion device (3; 3'; 3, 3'; 3, 3f, 3g), which is provided at least between two holes (4);
wherein the longitudinal expansion device (3; 3'; 3, 3'; 3, 3f, 3g) is designed in such a way that the plate implant (1b-1g) has increased local extensibility in the direction of the bone longitudinal axis (LA) with simultaneously high bending and torsional rigidity;
wherein the longitudinal expansion device (3; 3'; 3, 3'; 3, 3f, 3g) has a plurality of leaf springs (3; 3'; 3, 3'; 3, 3f, 3g), which are formed by one or more notches (5; 5', 5", 5"; 5; 5, 5e', 5e", 5e"'; 5, 5f', 5f"; 5g', 5g") in the plate implant (1b-g); and
wherein the leaf springs (3; 3'; 3, 3'; 3, 3f, 3g) are formed by U-shaped notches (5; 5', 5", 5"; 5; 5, 5e', 5e", 5e‴; 5, 5f', 5f"; 5g', 5g") in the plate implant (1b-1g), which surround a respective hole (4).

2. Plate implant (1b-1g) for bridging a fracture gap (6) according to claim 1, wherein a first plurality of notches (5f', 5f"; 5g', 5g") is designed in such a way that the U-shape is opened towards the left edge (LR) of the plate implant, and a second plurality of notches (5, 5f', 5f"; 5g', 5g") is designed in such a way that the U-shape is opened towards the right edge (RR) of the plate implant.

3. Plate implant (1b-1g) for bridging a fracture gap (6) according to claim 1, wherein the notches (5f', 5f"; 5g', 5g") overlap laterally.

4. Plate implant (1b-1g) for bridging a fracture gap (6) according to claim 1, wherein the leaf springs (3') are designed in such a way that they are formed by respective first and second notches (5', 5"), wherein the first and second notches (5") surround a respective hole (4) at least partially in a U-shaped manner, wherein the U-shape of the first notches (5') is open towards the left edge (LR) of the plate implant and the U-shape of the second notches (5") is open towards the right edge (RR) of the plate implant, and wherein other leaf springs (3') are formed by third notches (5‴), which are respectively arranged perpendicular to the longitudinal axis (LA) and are arranged between and at a distance from respective pairs of the first and second notches (5', 5").

5. Plate implant (1ba-g) for bridging a fracture gap (6) according to claim 4, wherein the second notches (5") are spaced apart opposite from the first notches (5'), and wherein the third notches (5‴) are respectively arranged perpendicular to the longitudinal axis (LA) between and at a distance from respective pairs of the first and second notches (5', 5").

6. Plate implant (1b-1g) for bridging a fracture gap (6) according to one of the preceding claims, **characterised by** a shaft region (R) with a substantially constant first width and a head region (K) continuously widening from the first width.

7. Plate implant (1g, 1g') according to claim 1, **characterised in that** the plate implant (1g, 1g') is designed individually and provided for reconstruction of a lower jaw (7) from fragments of a fibula.

8. Set of two plate implants (1g, 1g') according to claim 7 for reconstruction of a lower jaw (7) from fragments of the fibula.

## Revendications

1. Implant de type plaque (1b-1g) destiné à combler une fente de fracture (6), comprenant :
un axe longitudinal (LA) dans la direction de la plus grande extension de l'implant de type plaque (1b-1g) ;
une pluralité d'alésages (4) qui sont espacés les uns des autres dans la direction de l'axe longitudinal (LA) ; et
un dispositif d'extension longitudinale élastique (3 ; 3' ; 3, 3' ; 3, 3f ; 3g) qui est prévu au moins entre deux alésages (4) ;
dans lequel le dispositif d'extension longitudinale (3 ; 3' ; 3, 3' ; 3, 3f ; 3g) est conçu de telle sorte que l'implant de type plaque (1b-1g) présente une extensibilité locale accrue dans la direction de l'axe longitudinal de l'os (LA) tout en présentant une résistance élevée à la flexion et à la torsion ;
dans lequel le dispositif d'extension longitudinale (3 ; 3' ; 3, 3' ; 3, 3f ; 3g) présente plusieurs ressorts à lame (3 ; 3' ; 3, 3' ; 3, 3f ; 3g) qui sont formés par une ou plusieurs entailles (5 ; 5', 5", 5" ; 5 ; 5, 5e', 5e", 5e"' ; 5, 5f', 5f" ; 5g', 5g") dans l'implant de type plaque (1b-g) ; et
dans lequel les ressorts à lame (3 ; 3' ; 3, 3' ; 3, 3f ; 3g) sont formés par des entailles en forme de U (5 ; 5', 5", 5" ; 5 ; 5, 5e', 5e", 5e"' ; 5, 5f', 5f" ; 5g', 5g") dans l'implant de type plaque (1b-g), lesquelles entourent un alésage respectif (4).

2. Implant de type plaque (1b-g) destiné à combler une fente de fracture (6) selon la revendication 1, dans lequel une première pluralité d'entailles (5f', 5f" ; 5g', 5g") est conçue de telle sorte que la forme en U est ouverte vers le bord gauche (LR) de l'implant de type plaque, et une deuxième pluralité d'entailles (5, 5f', 5f" ; 5g', 5g") est conçue de telle sorte que la forme en U est ouverte vers le bord droit (RR) de l'implant de type plaque.

3. Implant de type plaque (1b-g) destiné à combler une fente de fracture (6) selon la revendication 1, dans lequel les entailles (5f', 5f" ; 5g', 5g") se chevauchent latéralement.

4. Implant de type plaque (1b-g) destiné à combler une fente de fracture (6) selon la revendication 1, dans lequel les ressorts à lame (3') sont conçus de telle sorte qu'ils sont formés par des première et deuxième entailles respectives (5', 5"), les première et deuxième entailles (5") entourant au moins partiellement en forme de U un alésage respectif (4), la forme en U des premières entailles (5') étant ouverte vers le bord gauche (LR) de l'implant de type plaque et la forme en U des deuxièmes entailles (5") étant ouverte vers le bord droit (RR) de l'implant de type plaque, et d'autres ressorts à lame (3') étant formés par des troisièmes entailles (5"') qui sont respectivement disposées perpendiculairement à l'axe longitudinal (LA) et qui sont disposées entre et à distance de paires respectives de premières et deuxièmes entailles (5', 5").

5. Implant de type plaque (1ba-g) destiné à combler une fente de fracture (6) selon la revendication 4, dans lequel les deuxièmes entailles (5") sont disposées à distance et en face des premières entailles (5'), et les troisièmes entailles (5"') sont respectivement disposées perpendiculairement à l'axe longitudinal (LA) entre et à distance de paires respectives de premières et deuxièmes entailles (5', 5").

6. Implant de type plaque (1b-g) destiné à combler une fente de fracture (6) selon l'une des revendications précédentes, **caractérisé par** une zone de corps (R) ayant une première largeur essentiellement constante et une zone de tête (K) s'élargissant de manière continue à partir de la première largeur.

7. Implant de type plaque (1g, 1g') selon la revendication 1, **caractérisé en ce que** l'implant de type plaque (1g, 1g') est conçu individuellement et est destiné à la reconstruction d'un maxillaire inférieur (7) à partir de fragments d'un péroné.

8. Ensemble de deux implants de type plaque (1g, 1g') selon la revendication 7 pour la reconstruction d'un maxillaire inférieur (7) à partir de fragments du péroné.
